# EUROPEAN PATENT SPECIFICATION

(11) **EP 4 608 260 B1**
(45) Date of publication and mention of the grant of the patent: **26.08.2026**
(21) Application number: 23783362.9
(22) Date of filing: 29.09.2023
(51) Int. Cl.: A61B 5/145, A61B 5/00

(54) **EPIDERMAL SUPPORT PATCH**
EPIDERMALES STÜTZPFLASTER
TIMBRE DE SUPPORT ÉPIDERMIQUE

(30) Priority: 26.10.2022 GB 202215850
(43) Date of publication of application: 03.09.2025
(73) Proprietor: Glucorx Technologies Limited, Dingwall IV15 9QF (GB)
(72) Inventor: MURRAY-SCOTT, Cerys Rohann, Dingwall IV15 9QF (GB)
(74) Representative: Marks & Clerk LLP
(86) International application number: PCT/EP2023/077158
(87) International publication number: WO 2024/088697

(56) References cited:
- WO-A1-2021/158372
- US-A1- 2017 188 912
- US-A1- 2021 378 592
- US-A1- 2022 202 366

## Description

### Field of Invention

The present disclosure relates to a device, kit of parts and method for securing a sensor module to the skin of a user, and to an epidermal support patch for use with such a kit of parts.

### Background

Traditionally, monitoring blood glucose levels of a patient involved a process where a finger prick blood test obtained a small drop of blood that was placed on a test strip that inserted into a glucometer. The glucometer read the strip and provided a digital reading of the individual's blood sugar level.

Recently, finger prick blood tests have been replaced by insertable (implantable), in vivo, analyte sensors that are inserted into the skin of the patient where they remain at all times, enabling substantially continuous measurements to be taken, which is advantageous compared to finger prick tests that provide only snapshot readings at a small number of times a day. These types of implantable analyte sensors are typically coupled to a sensor module having a housing placed on the surface of the patient's skin. For example, the rearwardly protruding part of the analyte sensor is inserted into the skin-facing surface of the housing the sensor module, where it is coupled inside the sensor module to control electronics. This ensures only the sensor module itself is visibly exposed to the outside environment and the entry point of the insertable analyte sensor in the skin is at least partially protected underneath the sensor module. The sensor module control electronics process measurement signals from the inserted sensor and transmit any relevant information to, for example, the patient's smartphone or other mobile device. These types of systems are sometimes known as continuous analyte monitoring systems.

Typically, continuous analyte monitoring systems require the analyte sensor to be replaced at predetermined intervals and this may require the patient to insert the analyte sensor themselves in an unsupervised environment. In order to simplify this process, known analyte monitoring systems are provided with an inserter device which applies a predetermined amount of force to the insertable analyte sensor to safely insert it into the patient's skin, and at the same time to position any accompanying sensor module at the surface of the patient's skin. EP2393417B1 proposes a continuous analyte monitoring system.

In known continuous analyte monitoring systems, such as that of EP2393417B1, the sensor module is initially inside of the inserter device. An adhesive pad or patch (sometimes referred to as an epidermal support patch) is provided on the skin-facing end of the inserter device. During use, the inserter device inserts the analyte sensor into the skin, places the sensor module onto the adhesive pad which is thereby secured to the patient's skin. The force of the sensor module and skin-facing rim of the inserter device during activation ensures the entire area of the adhesive pad is pressed securely onto the skin.

A problem of adhesive pads or patches for continuous analyte monitoring systems is that biocompatible adhesives used in such pads have a short shelf life. The short shelf life of the adhesive pads provided with the continuous analyte monitoring systems results in a short shelf life of the continuous analyte monitoring system as a whole because the patches are sterilised and included in a sterile condition already attached to skin-facing surface of the continuous analyte monitoring system. This requires warehouses and sellers to replace their stock of such monitoring systems regularly which is expensive and inconvenient. The features of the preambles of the independent claims are known from WO2021/158372.

An improved adhesive patch is desired.

### Summary

The present invention is defined in the claims.

According to present invention there is provided a kit of parts for securing a sensor module to the skin of a user, the kit of parts comprising:
a sensor module, an epidermal support patch, and an inserter device, the sensor module having a non-biocompatible adhesive on a surface thereof for securing the sensor module to the patch, the patch having a biocompatible adhesive on a surface thereof for securing the patch to the skin of a user; and the inserter device configured to, during use, move the sensor module into contact with the patch after the patch is secured to the skin of the user to secure the sensor module to the patch using said non-biocompatible adhesive,
wherein a footprint of the patch is at least 50% larger by area than a footprint of the inserter device.

In general terms, the present disclosure is directed to an epidermal support patch for kit of parts for securing a sensor module to the skin of a patient. Unlike in known systems, where the footprint of the patch matches or is smaller than the footprint of the inserter device because the inserter device is used to secure the patch to the skin, the epidermal support patch of the present disclosure has a footprint that is larger than the inserter device with which it is used (i.e. the outer perimeter of the patch is larger than the corresponding outer perimeter of the inserter device, in the plane of the patch).

The increased outer perimeter provides in an increased surface area, or footprint, of the patch (without increasing the footprint of the inserter device) and thus increases the amount of adhesive area secured to the patient's skin. This strengthens the bond between the patch and the skin, and thus reduces the likelihood that the patch, the analyte sensor and the sensor module will detach from the skin. The larger patch is also envisaged to be applied directly by the patient to their skin without relying on the inserter device to press the patch the skin. As a result, the size of the inserter device can be substantially smaller compared to known inserter devices for continuous analyte monitoring systems as it is no longer necessary to take adhesive surface area of the patch which the inserter device has to press on into account when designing the size of the inserter device.

Additionally, as the adhesive patch provided separately from the inserter device, its upper surface facing away from the skin may be coated at least partially with a non-biocompatible adhesive to secure the sensor module thereto. Non-biocompatible adhesives are typically substantially stronger than biocompatible adhesives and have a much longer shelf life.

As a result of the above described features, the inserter device, analyte sensor, and sensor module of the kit of parts may be provided in one item of packaging, sterilised and stored separately and for a much longer period of time than the patch without having to worry about a short shelf-life. Instead, the patches of the present disclosure are envisaged to be stored separately. Thus, if the biocompatible adhesive expires, it is only necessary to replace the stock of patches, rather than the entire continuous analyte monitoring system as is presently required for known continuous analyte monitoring systems.

Thus, according to a first aspect of the disclosure, there is provided a kit of parts for securing a sensor module to the skin of a user, the kit of parts comprising: a sensor module, an epidermal support patch, and an inserter device, the sensor module having a non-biocompatible adhesive on a surface thereof for securing the sensor module to the patch, the patch having a biocompatible adhesive on a surface thereof for securing the patch to the skin of a user; and the inserter device configured to, during use, move the sensor module into contact with the patch after the patch is secured to the skin of the user to secure the sensor module to the patch using said non-biocompatible adhesive, wherein an outer perimeter of the patch is larger than an outer perimeter of the inserter device.

Optionally, an exposed surface of the patch defines a raised portion at least partially surrounding a central area of the exposed surface of the patch.

Advantageously, the raised portion, for example a circular ring-like structure around the exposed surface of the patch (i.e. the surface facing away from the skin), acts as a guide to ensure correct placement of the inserter device by the user as close to being centred on the patch as possible.

Optionally central area has a perimeter corresponding substantially (for example in shape and/or length) to a perimeter of a portion the inserter device in contact with the patch during use.

Advantageously, matching the perimeter of the portion of the inserter device that touches the patch to the perimeter of the central area defined by the raised portion provides a passive haptic feedback effect where the user can immediately feel if the inserter device is positioned correctly as it slots directly into the central area defined by the raised portion. Where the perimeter of the central corresponds substantially to that of the portion of the inserter device, it is envisaged that the raised portion may thus be just larger than that perimeter so that the inserter device portion can fit in the central area for example snugly.

Optionally, the inserter device comprises a body housing a force applicator, a carriage for receiving the sensor module therein before use, and a carriage release sleeve, the carriage release sleeve being configured to release the carriage under a force applied by the force applicator, thereby moving the sensor module into contact with the patch, and wherein said portion of the inserter device in contact with the patch during use comprises said carriage release sleeve.

Optionally, the raised portion is configured to guide placement of the inserter device onto a predetermined position on the exposed surface of the patch in the central area during use of the inserter device

Advantageously, it is envisaged that the inserter device is activated by pressing the carriage release sleeve onto the patient's skin rather than requiring a button or other activation mechanism, thereby simplifying the internal structure of the inserter device.

Optionally, in this case, the raised portion is configured to guide placement of the inserter device, and particularly the carriage release sleeve, onto a predetermined position on the exposed surface of the patch in the central area during use of the inserter device.

Thus, as described above, the user is provided with a passive haptic feedback feeling as a result of sliding the forward facing carriage release sleeve of the inserter device over the raised potion until it fits when the carriage release sleeve is correctly placed in the predetermined position.

Optionally, the sensor module comprises an analyte sensor having at least one sharp end, and wherein, during use, the inserter device is configured to drive the sharp end of the analyte sensor at least partially into the skin of the user.

As will be appreciated, the inserter device may be provided with a hollow needle, or other sharp configured to hold the analyte sensor as it is inserted by the inserter device into the skin. Such mechanisms are known to the skilled person and any suitable such mechanism may be provided.

Optionally, the analyte sensor is a blood glucose sensor.

Whilst it is envisaged that the analyte sensor of the present disclosure is a blood glucose sensor. It is also envisaged that the sensor may be used to measure other analytes.

Optionally, the sensor module is configured to be secured to the skin of the user with the patch without an over-patch.

In known continuous analyte systems, an over-patch is typically required to ensure the analyte sensor and sensor module remain safely secured to the skin. Such an over-patch is typically a large patch of adhesive configured to be placed on top of the sensor module. This is inconvenient for the patient. Thus, advantageously, a patch having a larger footprint than that of the inserter device can have a much greater adhesive area and remain secure on the patient without requiring an over-patch.

As will be appreciated, a patch with a larger footprint than the footprint of the inserter device may also be defined in other ways, as set out below.

For example, optionally, patch has diameter or lateral extent in the plane of the skin larger than a diameter or lateral extent of the inserter device in the plane of the skin during use.

For example, optionally, the outer perimeter of the patch defines a footprint of the patch in the plane of the skin or patch, wherein the outer perimeter of the inserter device defines a footprint of the inserter device in the plane of the skin or patch during use, and wherein the footprint of the patch is larger than the footprint of the inserter device.

Optionally, it is envisaged that said perimeter, or where applicable, diameter, lateral extent, or footprint of the patch is at least 20%-100% larger than said corresponding perimeter, or where applicable, diameter, lateral extent, or footprint of the inserter device, for example 30%, 40%, 50%, 60%, 70%, 80%, or 90%. For example, it is found that a patch having a footprint around 50% larger in area than that of the inserter device provides ideal increased adhesive strength relative to known systems.

According to a second aspect of the disclosure, there is provided, a method of securing a sensor module to the skin of a user, the method comprising: securing a surface of an epidermal support patch having a biocompatible adhesive thereon to the skin of a user; with an inserter device, moving a sensor module into contact with the patch; and securing a surface of the sensor module having a non-biocompatible adhesive thereon to the patch, wherein a footprint of the patch is larger than a footprint of the inserter device.

Optionally, an exposed surface of the patch defines a raised portion at least partially surrounding a central area of the exposed surface of the patch.

Optionally, the central area has a perimeter corresponding substantially (for example in shape and/or length) to a perimeter of a portion the inserter device in contact with the patch during use.

Optionally, the step of moving the sensor module into contact with the patch comprises aligning said portion of the inserter device with the raised portion.

The advantages described above in connection with the corresponding features of the first aspect also apply to the second aspect of the disclosure.

According to a third aspect of the disclosure, there is provided an epidermal support patch for securing a sensor module to the skin of a patient, the patch comprising a biocompatible adhesive on a first surface thereof, wherein a second surface of the patch defines a raised portion at least partially surrounding a central area of the second surface of the patch.

According to a fourth aspect of the disclosure, there is provided an inserter device for securing a sensor module to the skin of a user, the inserter device configured, during use, to drive a pointed end of the sensor module at least partially into the skin of the user, the inserter device comprising:
a body housing a carriage for receiving the sensor module therein before use;
a carriage release sleeve comprising one or more deformable sections configured to engage one or more corresponding holding abutments on an inner surface of the body;
wherein the carriage release sleeve is configured to release the carriage under a force upon sufficient relative movement of the carriage release sleeve with the body.

Optionally, the one or more deformable sections may comprise deformable wall sections, and the relative movement of the carriage release sleeve with the body may cause the one or more holding abutments to push the deformable wall sections radially inwards, the radially inward movement of the deformable wall sections configured to release the carriage from the carriage release sleeve.

The deformable wall sections may be configured to release the carriage from the carriage release sleeve at an end of an inward stroke of the body relative to the carriage release sleeve.

Optionally, the one or more holding abutments are cam surfaces.

Optionally, the inserter device may comprise a force applicator, the force applicator configured to apply a force from the body to the carriage. The force applicator may be a spring or any other force generating mechanism.

Optionally, the carriage may comprise a protrusion configured, during use, to guide the at least one pointed end of the sensor module to pierce the skin of the user after the carriage is released from the carriage release sleeve. The protrusion may be a cannula, and the inserter device may comprise an automatic cannula retraction mechanism.

Optionally, the inner surface of the body may comprise one or more guide rails for guiding a movement path of the carriage release sleeve within the body, the one or more guide rails configured to engage with one or more corresponding abutment surfaces on a surface of the carriage release sleeve.

The advantages described above in connection with the corresponding features of the first aspect also apply to the third aspect of the disclosure.

### Brief Description of the Drawings

These and other aspects will now be described, by way of example only, with reference to the accompanying figures in which:
Figure 1a illustratively shows a side view of a kit of parts according to the present disclosure.
Figure 1b illustratively shows an alternative view of the kit of parts of Figure 1a.
Figure 2a illustratively shows an exploded of an inserter device according to the present disclosure.
Figure 2b illustratively shows an assembled view of the inserter device of Figure 2a.
Figure 3a illustratively shows an epidermal support patch according to the present disclosure.
Figure 4 illustrates steps of a method according to the present disclosure.

### Detailed Description

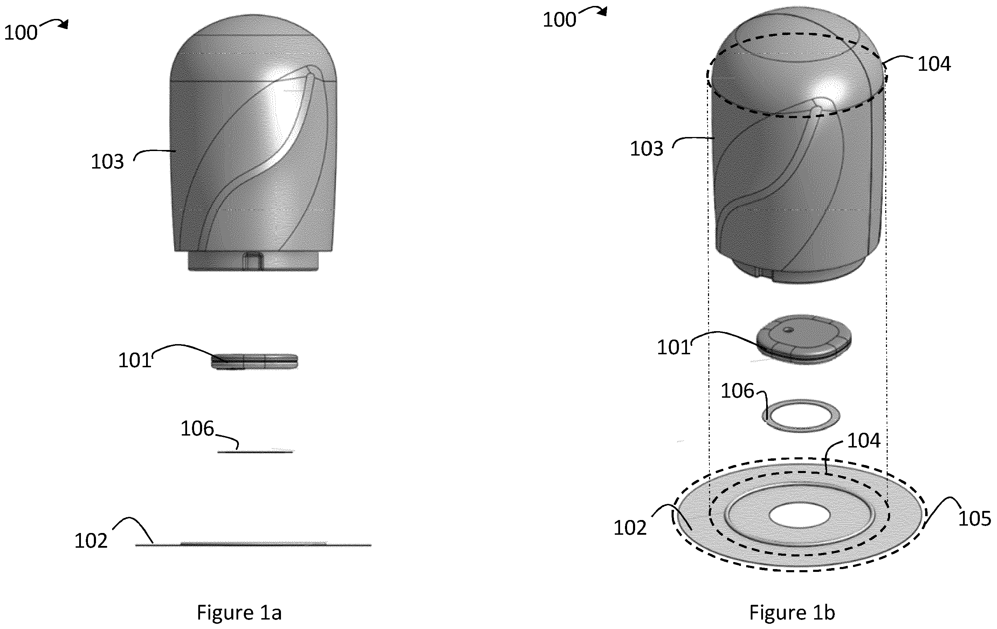

Figure 1a and 1b illustratively show two views of a kit of parts 100 according to the present disclosure. The kit of parts 100 comprises a sensor module 101, an epidermal support patch 102, and an inserter device 103. During use, the kit of parts 100 is configured to secure the sensor module 101 to the skin of a user, for example a patient who uses the sensor module 101 to make continuous blood glucose readings outside of a clinical environment.

The sensor module 101 has a non-biocompatible adhesive 106 on a surface thereof facing towards the user's skin. The non-biocompatible adhesive 106 is configured for securing the sensor module 101 to an exposed surface of the epidermal support patch 102. Example non-biocompatible adhesives include, for example, cyanoacrylate based adhesives, urethane based adhesives, and phenoxyethoxy ethylacrylate adhesives. These non-biocompatible adhesives 106 have a stronger bond strength and substantially longer shelf lives compared to typical biocompatible adhesives so may accordingly be sterilised and packaged with, for example, the sensor module and/or inserter device and thereby provide a much longer shelf life compared to kits where a biocompatible adhesive is included therewith as an integral part.

The patch 102 has a biocompatible adhesive on a skin-facing surface thereof for securing the patch 102 to the skin of the user, leaving the opposite facing surface exposed and ready to receive the surface of the sensor module 101 having the non-biocompatible adhesive 101 thereon. Example biocompatible adhesives include, for example, Infinity Bond Medical Device Super Glue Cyanoacrylate, LOCTITE ^{™} adhesives, DuPont^{™} Liveo^{™} BIO-PSA Amine-Compatible Silicone Adhesive. Other suitable biocompatible adhesives will also be known to the skilled person. The patch 102 accordingly provides a shield layer between the non-biocompatible adhesive 106 of the sensor module 101 and the skin of the user to protect the skin from the non-biocompatible adhesive of the sensor module 101. It is envisaged that, during use of the kit of parts, the user first manually applies the patch to their skin, securing it by pressing down over its entire exposed area with their hands or fingers and only once it is secured on the user's skin is the inserter device used to secure the sensor module on the patch.

Thus, the inserter device 103 is configured to, during use, move the sensor module 101 into contact with the patch 102 after the patch 102 is secured to the skin of the user to secure the sensor module 101 to the patch 102 using said non-biocompatible adhesive.

As the patch 102 is not secured to the skin with the inserter device 103, the inserter device 103 may be smaller than the patch 102, thereby providing a more convenient kit of parts compared to known systems where the patch is substantively the same size (for example in perimeter, area, diameter, footprint, or other corresponding dimension as described below).

In Figure 1b, the footprint (i.e. outer perimeter) 104 of the inserter device 103 and the footprint (i.e. outer perimeter) 105 of the patch 102 are illustrated with dashed lines. As can be seen in Figure 1b, the outer perimeter 105 of the patch 102 is larger (i.e. extend to a greater radius relative to the centre of the patch 102) around its entire circumference compared to the outer perimeter 104 of the inserter device 103. This may also be described in other ways, for example, where the patch 102 and inserter device 103 are circular, the diameter of the patch 102 is greater than the diameter of the inserter device 103. For example, the inserter device 103 may be said to have a footprint (i.e. extent of the shape of the inserter device 103 projected onto or in contact with the surface of the skin) and this footprint is smaller than the footprint of the patch 102, for example having a smaller area.

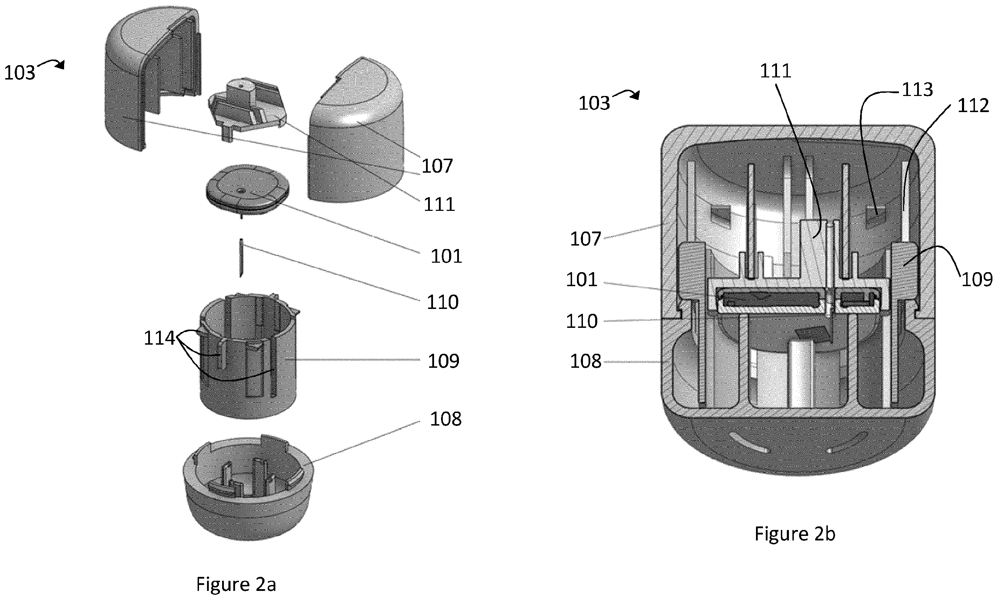

Figures 2a and 2b illustratively show an inserter device 103 according to the present disclosure, for example similar to the inserter device corresponding to that of Figures 1a and 1b, but with a different overall body shape. The inserter device 103 comprises a body 107 and a removable cap 108 covering a forward end thereof (the term forward referring to the direction that faces the user's skin during use as opposed to rearward which refers to the opposite direction).

Inside the space enclosed by the body 107 and removable cap 108, the inserter device 103 further comprises a carriage 111 for receiving the sensor module 101 therein before use, and a carriage release sleeve 109 configured to release the carriage 111 upon relative movement of the carriage release sleeve 109 with the body 107.

The carriage 111 is provided with a cannula 110 or other corresponding sharp configured to hold and/or guide the analyte sensor of the sensor module 101 and pierce the skin as the carriage 111 with sensor module 101 is driven forwards to implant or insert the sensor into the user's skin.

The internal surface of the body 107 may be provided with one or more guide rails 112 and/or hooks, recesses abutment surfaces 113 to guide the movement path of the carriage release sleeve 109 within the body by engagement with one or more corresponding slots, hooks, recesses, cam surfaces and/or abutment surfaces 114 on a surface of the carriage release sleeve 109.

For example, the carriage release sleeve 109 may be provided with one or more deformable wall sections with hooks that engage one or more corresponding a cam surface on the inner surface of the body 107. When a user presses the inserter device onto the patch 102 on their skin after removing the cap 108, the carriage release sleeve 109 contacts the surface of the patch 102 and, as the user continues to press, the body 107 moves forwards relative to the carriage release sleeve 109 causing the one or more cam surfaces to push deformable wall sections radially inwards until the end of the inward stroke is reached.

Alternatively, instead of a manual activation, the inserter device 103 may further comprise an optional force applicator (not shown) such as a spring or other force generating mechanism, coupled to the carriage 111. Pressing the carriage release sleeve onto the patch 102 forces one or more hooks from one or more holding abutments inside the body 107 thereby releasing the carriage forward under the force of the force applicator and causing the sensor to be implanted into the skin.

The inserter device 103 shown in Figures 2a and 2b is not provided with an automatic cannula retraction mechanism. Instead, the inserter device 103 is manually lifted up after use, thereby pulling the cannula 110 out of the skin but leaving the analyte sensor implanted in the skin. However, it is also envisaged that an automatic cannula retraction mechanism may be provided, as will be appreciated by the skilled person.

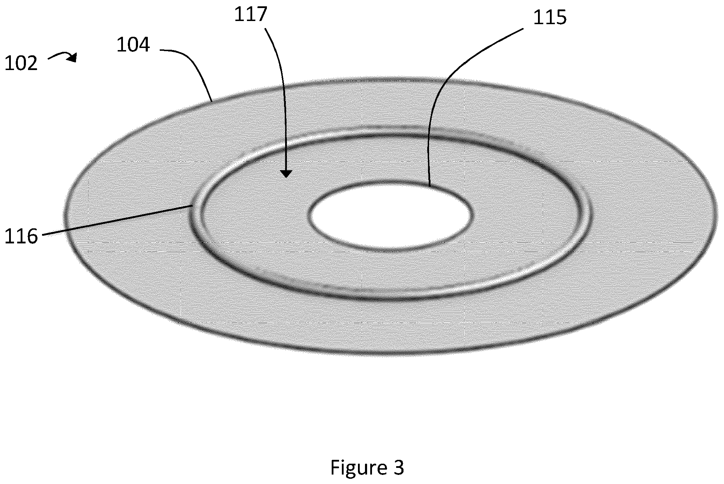

Figure 3 illustratively shows an epidermal support patch 102 according to the present disclosure. The patch 102 has circular with an outer perimeter 104 and an inner perimeter 115 defining a central hole through which it is envisaged the cannula and analyte sensor described above are inserted into the skin.

The surface of the patch 103 visible in Figure 3 is exposed after it is applied to the skin. The surface on the opposite side is provided with a biocompatible adhesive configured to secure the patch 102 to the user's skin. The exposed surface defines a raised portion 116 at least partially surrounding a central area 117 of the patch 102. The raised portion 116 has a ring like shape. It is envisaged the raised portion is integral with the patch, for example made of the same or similar material as the patch 102, or alternatively made of a different material to the patch, for example a material that is harder than the patch 102, as opposed to being a pre-affixed sensor module unit or other such housing. The raised portion 116, and accordingly the central area 117 defined thereby, has a perimeter corresponding substantially to the perimeter of the carriage release sleeve 109 of the inserter device 103 i.e. the portion of the inserter device 103 that is in contact with the patch 102 during use. Thus, the raised portion 116 acts as a guide to position the inserter device 103 correctly and centrally on the patch to ensure correct placement thereof onto the patch. In contrast to any known patches without a raised portion which require the patch to be pre-affixed to an inserter device in a correct position before use, for example by the manufacturer using high precision manufacturing techniques, the present patch may be applied at any time prior to use to the patient's skin and the raised portion used to ensure correct placement of the inserter device at any time thereafter.

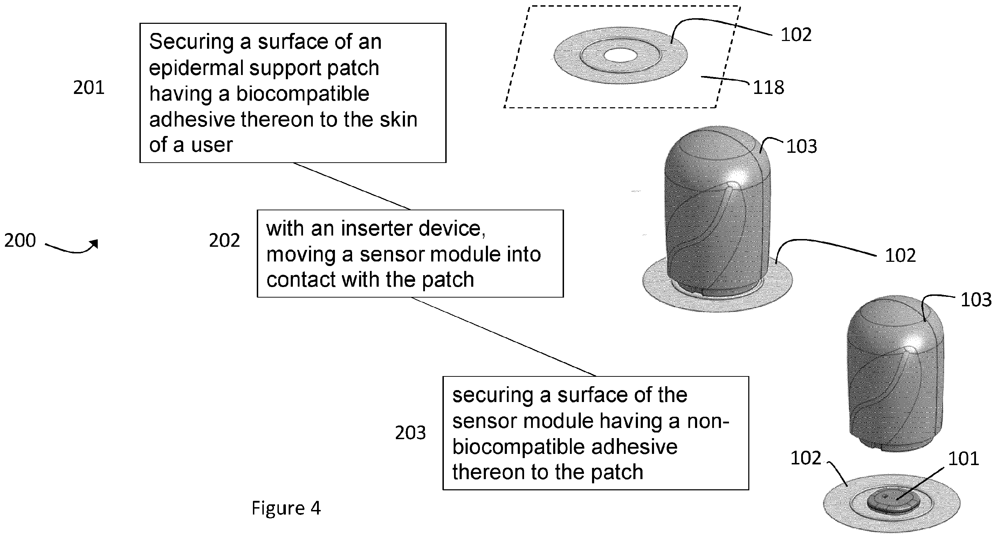

Figure 4 illustrates steps of a method 200 according to the present disclosure of securing a sensor module to the skin of a user. The method 200 comprises: securing 201 a surface of an epidermal support patch 102 having a biocompatible adhesive thereon to the skin 118 of a user, with an inserter device 103, moving 202 a sensor module 101 into contact with the patch 102; and securing 203 a surface of the sensor module 101 having a non-biocompatible adhesive thereon to the patch 101. The outer perimeter of the patch 102 is larger than an outer perimeter of the inserter device 103.

Other effective alternatives will occur to the skilled person. It will be understood that the invention is not limited to the described embodiments and encompasses modifications apparent to those skilled in the art lying within the scope of the claims appended hereto.

For example, the whilst a circular patch with a ring-shaped raised portion is envisaged, other shapes are also possible including, but not limited to, square or rectangular or hexagonal shapes. In each case, it is envisaged that substantially the whole surface outside of the central area of the patch is exposed while the inserter device is placed thereon, thus providing a substantially increased surface area with adhesive thereon in contact with the patient's skin compared to patches substantially wholly covered by an inserter device during its use.

For example, whilst the above kit of parts is described in the context of blood glucose analyte sensors, it is envisaged that the kit of parts may be used for any analyte whose measurement may be made by an implantable continuous analyte sensor, including for example, lactate, b-hydroxybutyrate, ethanol, cholesterol, and/or uric acid.

## Claims

1. A kit of parts (100) for securing a sensor module to the skin of a user, the kit of parts comprising:
a sensor module (101), an epidermal support patch (102), and an inserter device (103), the sensor module having a non-biocompatible adhesive (106) on a surface thereof for securing the sensor module to the patch, the patch having a biocompatible adhesive on a surface thereof for securing the patch to the skin of a user; and the inserter device configured to, during use, move the sensor module into contact with the patch after the patch is secured to the skin of the user to secure the sensor module to the patch using said non-biocompatible adhesive,
**characterized in that** a footprint (104) of the patch is at least 50% larger by area than a footprint of the inserter device.

2. The kit of parts according to claim 1, wherein an exposed surface of the patch defines a raised portion (116) at least partially surrounding a central area (117) of said exposed surface of the patch.

3. The kit of parts according to claim 2, wherein the central area has a perimeter corresponding substantially to a perimeter of a portion the inserter device in contact with the patch during use.

4. The kit of parts according to claim 3, wherein the inserter device comprises a body (107) housing a carriage (111) for receiving the sensor module therein before use, and a carriage release sleeve (109), the carriage release sleeve being configured to release the carriage under a force, thereby moving the sensor module into contact with the patch, and
wherein said portion of the inserter device in contact with the patch during use comprises said carriage release sleeve.

5. The kit of parts according to any of claims 2 to 4, wherein the raised portion is configured to guide placement of the inserter device onto a predetermined position on the exposed surface of the patch in the central area during use of the inserter device.

6. The kit of parts according to any preceding claim, wherein the sensor module comprises an analyte sensor having at least one pointed end, and wherein, during use, the inserter device is configured to drive the pointed end of the analyte sensor at least partially into the skin of the user.

7. The kit of parts according to any preceding claim, wherein the analyte sensor is a blood glucose sensor.

8. The kit of parts according to any preceding claim, wherein the sensor module is configured to be secured to the skin of the user with the patch without an over-patch.

9. The kit of parts according to any preceding claim, wherein the patch has diameter or lateral extent in the plane of the skin larger than a diameter or lateral extent of the inserter device in the plane of the skin during use.

10. The kit of parts according to any preceding claim, wherein said footprint is defined by an outer perimeter of the patch in the plane of the skin, wherein said footprint of the inserter device is defined by an outer perimeter of the inserter device in the plane of the skin during use.

11. A method (200) of securing a sensor module (101) to the skin of a user, the method comprising:
securing (201) a surface of an epidermal support patch (102) having a biocompatible adhesive (106) thereon to the skin of a user;
with an inserter device (103), moving (202) a sensor module into contact with the patch; and
securing (203) a surface of the sensor module having a non-biocompatible adhesive thereon to the patch,
**characterized in that** a footprint (104) of the patch is at least 50% larger by area than a footprint of the inserter device.

12. The method of claim 11, wherein an exposed surface of the patch defines a raised portion (116) at least partially surrounding a central area (117) of the exposed surface of the patch.

13. The method of claim 12, wherein the central area has a perimeter corresponding substantially to a perimeter of a portion the inserter device in contact with the patch during use.

14. The method of claim 13, wherein said step of moving the sensor module into contact with the patch comprises aligning said portion of the inserter device with the raised portion.

## Patentansprüche

1. Teilesatz (100) zum Befestigen eines Sensormoduls auf der Haut eines Benutzers, wobei der Teilesatz Folgendes umfasst:
ein Sensormodul (101), ein epidermales Stützpflaster (102) und eine Einführvorrichtung (103), wobei das Sensormodul auf einer der Oberflächen davon einen nicht biokompatiblen Klebstoff (106) zum Befestigen des Sensormoduls am Pflaster aufweist, wobei das Pflaster auf einer Oberflächen davon einen biokompatiblen Klebstoff zum Befestigen des Pflasters auf der Haut eines Benutzers aufweist; und die Einführvorrichtung so konfiguriert ist, dass sie während Verwendung das Sensormodul, nachdem das Pflasters auf der Haut des Benutzers befestigt ist, in Kontakt mit dem Pflaster bewegt, um das Sensormodul unter Verwendung des nicht biokompatiblen Klebstoffs am Pflaster zu befestigen,
**dadurch gekennzeichnet, dass**
eine Grundfläche (104) des Pflasters flächenmäßig mindestens 50 % größer als die Grundfläche der Einführvorrichtung ist.

2. Teilesatz nach Anspruch 1, wobei eine freiliegende Oberfläche des Pflasters einen erhöhten Abschnitt (116) definiert, der mindestens teilweise einen zentralen Bereich (117) der freiliegenden Oberfläche des Pflasters umschließt.

3. Teilesatz nach Anspruch 2, wobei der zentrale Bereich einen Umfang aufweist, der im Wesentlichen einem Umfang eines Abschnitts der Einführvorrichtung entspricht, der während Verwendung mit dem Pflaster in Kontakt ist.

4. Teilesatz nach Anspruch 3, wobei die Einführvorrichtung einen Körper (107) umfasst, in dem ein Schlitten (111) zum Aufnehmen des Sensormoduls vor Verwendung untergebracht ist, und eine Schlittenfreigabehülse (109), wobei die Schlittenfreigabehülse so konfiguriert ist, dass sie den Schlitten unter einer Kraft freigibt und dadurch das Sensormodul mit dem Pflaster in Kontakt bewegt, und
wobei der Abschnitt der Einführvorrichtung, der während Verwendung mit dem Pflaster in Kontakt ist, die Schlittenfreigabehülse umfasst.

5. Teilesatz nach einem der Ansprüche 2 bis 4, wobei der erhöhte Abschnitt so konfiguriert ist, dass er in Verwendung Platzierung der Einführvorrichtung auf einer vorbestimmten Position auf der freiliegenden Oberfläche des Pflasters im zentralen Bereich der Einführvorrichtung leitet.

6. Teilesatz nach einem vorstehenden Anspruch, wobei das Sensormodul einen Analytsensor umfasst, der mindestens ein spitzes Ende aufweist, und wobei die Einführvorrichtung während Verwendung so konfiguriert ist, dass sie das spitze Ende des Analytsensors mindestens teilweise in die Haut des Benutzers eintreibt.

7. Teilesatz nach einem vorstehenden Anspruch, wobei es sich bei dem Analytsensor um einen Blutzuckersensor handelt.

8. Teilesatz nach einem vorstehenden Anspruch, wobei das Sensormodul so konfiguriert ist, dass es mit dem Pflaster ohne zusätzliches Überpflaster auf der Haut des Benutzers befestigt werden kann.

9. Teilesatz nach einem vorstehenden Anspruch, wobei das Pflaster einen Durchmesser oder eine seitliche Ausdehnung in der Ebene der Haut aufweist, die während Verwendung größer ist als ein Durchmesser oder seitliche Ausdehnung der Einführvorrichtung in der Ebene der Haut.

10. Teilesatz nach einem vorstehenden Anspruch, wobei die Grundfläche durch einen äußeren Umfang des Pflasters in der Ebene der Haut definiert ist, wobei die Grundfläche der Einführvorrichtung während Verwendung durch einen äußeren Umfang der Einführvorrichtung in der Ebene der Haut definiert ist.

11. Verfahren (200) zum Befestigen eines Sensormoduls (101) auf der Haut eines Benutzers, wobei das Verfahren Folgendes umfasst:
Befestigen (201) einer Oberfläche eines epidermalen Stützpflasters (102), die einen biokompatiblen Klebstoff (106) darauf aufweist, auf der Haut eines Benutzers;
mit einen Einführvorrichtung (103), Bewegen (202) eines Sensormoduls in Kontakt mit dem Pflaster; und
Befestigen (203) einer Oberfläche des Sensormoduls, die einen nicht biokompatiblen Klebstoff darauf aufweist, an dem Pflaster,
**dadurch gekennzeichnet, dass**
eine Grundfläche (104) des Pflasters flächenmäßig mindestens 50 % größer als die Grundfläche der Einführvorrichtung ist.

12. Verfahren nach Anspruch 11, wobei eine freiliegende Oberfläche des Pflasters einen erhöhten Abschnitt (116) definiert, der mindestens teilweise einen zentralen Bereich (117) der freiliegenden Oberfläche des Pflasters umschließt.

13. Verfahren nach Anspruch 12, wobei der zentrale Bereich einen Umfang aufweist, der im Wesentlichen einem Umfang eines Abschnitts der Einführvorrichtung entspricht, der während Verwendung mit dem Pflaster in Kontakt ist.

14. Verfahren nach Anspruch 13, wobei der Schritt des Bewegens des Sensormoduls in Kontakt mit dem Pflaster Ausrichten des Abschnitts der Einführvorrichtung mit dem erhöhten Abschnitt umfasst.

## Revendications

1. Kit de pièces (100) pour la fixation d'un module capteur sur la peau d'un utilisateur, ce kit de pièces comprenant :
un module capteur (101), un patch de support épidermique (102) et un dispositif d'insertion (103), le module capteur comportant un adhésif non biocompatible (106) sur une de ses surfaces pour le fixer au patch, le patch comportant un adhésif biocompatible sur une de ses surfaces pour le fixer à la peau de l'utilisateur ; et le dispositif d'insertion étant configuré pour, pendant l'utilisation, déplacer le module capteur en contact avec le patch après que celui-ci a été fixé à la peau de l'utilisateur afin de fixer le module capteur au patch à l'aide dudit adhésif non biocompatible,
**caractérisé en ce que**
une empreinte (104) du patch est au moins 50 % plus grande en surface qu'une empreinte du dispositif d'insertion.

2. Kit de pièces selon la revendication 1, dans lequel une surface exposée du patch définit une partie surélevée (116) entourant au moins partiellement une zone centrale (117) de ladite surface exposée du patch.

3. Kit de pièces selon la revendication 2, dans lequel la zone centrale présente un périmètre correspondant sensiblement au périmètre d'une partie du dispositif d'insertion en contact avec le patch pendant son utilisation.

4. Kit de pièces selon la revendication 3, dans lequel le dispositif d'insertion comprend un corps (107) abritant un chariot (111) destiné à recevoir le module capteur à l'intérieur avant utilisation, et un manchon de libération de chariot (109), le manchon de libération de chariot étant configuré pour libérer le chariot sous l'effet d'une force pour déplacer le module capteur en contact avec le patch, et
dans lequel ladite partie du dispositif d'insertion en contact avec le patch pendant l'utilisation comprend ledit manchon de libération du chariot.

5. Kit de pièces selon l'une quelconque des revendications 2 à 4, dans lequel la partie surélevée est configurée pour guider le placement du dispositif d'insertion sur une position prédéterminée sur la surface exposée du patch dans la zone centrale lors de l'utilisation du dispositif d'insertion.

6. Kit de pièces selon une quelconque revendication précédente, dans lequel le module capteur comprend un capteur d'analyte doté d'au moins une extrémité pointue, et dans lequel, lors de l'utilisation, le dispositif d'insertion est configuré pour enfoncer au moins partiellement l'extrémité pointue du capteur d'analyte dans la peau de l'utilisateur.

7. Kit de pièces selon une quelconque revendication précédente, dans lequel le capteur d'analyte est un capteur de glycémie.

8. Kit de pièces selon une quelconque revendication précédente, dans lequel le module capteur est configuré pour être fixé à la peau de l'utilisateur avec le patch sans sur-patch.

9. Kit de pièces selon une quelconque revendication précédente, dans lequel le patch présente un diamètre ou une étendue latérale dans le plan de la peau supérieure au diamètre ou à l'étendue latérale du dispositif d'insertion dans le plan de la peau pendant son utilisation.

10. Kit de pièces selon une quelconque revendication précédente, dans lequel ladite empreinte est définie par un périmètre extérieur du patch dans le plan de la peau, dans lequel ladite empreinte du dispositif d'insertion est définie par un périmètre extérieur du dispositif d'insertion dans le plan de la peau pendant l'utilisation.

11. Procédé (200) de fixation d'un module capteur (101) sur la peau d'un utilisateur, le procédé comprenant :
la fixation (201) d'une surface d'un patch de support épidermique (102) doté d'un adhésif biocompatible (106) sur la peau d'un utilisateur ;
avec un dispositif d'insertion (103), le déplacement (202) d'un module de capteur en contact avec le patch ; et
la fixation (203) d'une surface du module capteur comportant un adhésif non biocompatible sur le patch,
**caractérisé en ce que**
une empreinte (104) du patch est au moins 50 % plus grande en surface qu'une empreinte du dispositif d'insertion.

12. Procédé selon la revendication 11, dans lequel une surface exposée du patch définit une partie surélevée (116) entourant au moins partiellement une zone centrale (117) de la surface exposée du patch.

13. Procédé selon la revendication 12, dans lequel la zone centrale présente un périmètre correspondant sensiblement au périmètre d'une partie du dispositif d'insertion en contact avec le patch pendant son utilisation.

14. Procédé selon la revendication 13, dans lequel ladite étape de déplacement du module de capteur en contact avec le patch comprend l'alignement de ladite partie du dispositif d'insertion avec la partie surélevée.
